(19) Europäisches Patentamt
European Patent Office
Office européen des brevets

(11) Veröffentlichungsnummer: **0 303 892 A1**

# (12) EUROPÄISCHE PATENTANMELDUNG

(21) Anmeldenummer: **88112588.4**

(51) Int. Cl.⁴: **C07D 251/50**

(22) Anmeldetag: **03.08.88**

(30) Priorität: **15.08.87 DE 3727253**

(43) Veröffentlichungstag der Anmeldung: **22.02.89 Patentblatt 89/08**

(84) Benannte Vertragsstaaten: **CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Jäger, Horst, Dr.**
**Carl-Rumpff-Strasse 37**
**D-5090 Leverkusen 1(DE)**

## (54) Verfahren zur Monoacylierung wasserlöslicher organischer Aminoverbindungen.

(57) Ein verbessertes Verfahren zur Monoacylierung wasserlöslicher organischer Amine mit 2,4,6-Trifluortriazin in wäßrigem Medium besteht darin, daß man die Reaktion in Gegenwart anorganischer und/oder organischer wasserlöslicher Salze und/oder wassermischbarer organischer Lösungsmittel bei Temperaturen unter 0 °C durchführt.

EP 0 303 892 A1

## Verfahren zur Monoacylierung wasserlöslicher organischer Aminoverbindungen

Die Monoacylierung von wasserlöslichen organischen Aminoverbindungen mit 2,4,6-Trifluortriazin (Cyanurfluorid) wird im allgemeinen so durchgeführt, daß zu einer Aminoverbindung in wäßrigem Medium Cyanurfluorid zudosiert und dabei durch Zugabe von Eis oder Kühlung von außen eine Temperatur von 0 bis 5° eingehalten wird (vgl. DE-OS 27 46 109, EP-A 0 172 790, DE-OS 27 47 011, DE-OS 29 03 594). Diese Kondensation verläuft vielfach uneinheitlich, da infolge der hohen Reaktivität der primär entstandenen Monokondensationsprodukte des Cyanurfluorids diese sofort mit noch vorhandener nicht acylierter Aminoverbindung zu Dikondensationsprodukten weiterreagieren.

Gegenstand der vorliegenden Erfindung ist nun die Monoacylierung von wasserlöslichen organischen Aminen mit 2,4,6-Trifluortriazin in wäßrigem Medium bei Temperaturen unter 0°C in Gegenwart anorganischer und/oder organischer wasserlöslicher Salze und/oder wassermischbarer organischer Lösungsmittel.

Das Verfahren wird vorzugsweise so durchgeführt, daß man das Amin in wäßrigem Medium vorlegt und das Triazin unter 0°C zudosiert.

Das Verfahren kann auch als kontinuierliches Verfahren durchgeführt werden, beispielsweise in der Weise, daß man Amin und Triazin in der für den gewünschten Durchsatz erforderlichen Menge gleichzeitig und kontinuierlich in den Reaktionsraum leitet und die gebildeten Reaktionsprodukte kontinuierlich aus dem Reaktionsraum entfernt (vgl. DE-OS 27 46 109) oder daß man Amin und Triazin gleichzeitig und kontinuierlich in einen ersten Reaktor leitet, dort eine intensive Durchmischung vornimmt und die Reaktionsmischung anschließend in einem zweiten Reaktor leitet in dem nur geringe Rückvermischung aber gute radiale Vermischung eintritt und dort die Umsetzung zu Ende führt (vgl. EP 172 790).

Besonders geeignete wäßrige Medien sind dabei Gemische aus Wasser und anorganischen bzw. organischen Salzen bzw. wasserlöslichen organischen Lösungsmitteln.

Die bei der Reaktion freiwerdende Flußsäure kann in üblicher Weise mit alkalisch reagierenden Verbindungen neutralisiert werden.

Triazin und Amin werden im allgemeinen im Molverhältnis 0,8:1 bis 1,5:1, vorzugsweise 1:1 bis 1,2:1, insbesondere 1,1:1 bis 1,08:1 zur Reaktion gebracht.

Die Amine liegen vorzugsweise in Lösung vor und werden vorzugsweise in Form ihrer neutralen Alkalisalze eingesetzt. Das erfindungsgemäße Verfahren eignet sich vorzugsweise für die Acylierung chromophorer wasserlöslicher organischer Amine, insbesondere die aus der Chemie der Reaktivfarbstoffe bekannten aminogruppenhaltigen Azo-, Anthrachinon-, Phthalocyanin-, Formazan- oder Dioxazinfarbstoffe.

Das Verfahren eignet sich insbesondere auch für die Acylierung nicht-chromophorer wasserlöslicher organischer Amine, insbesondere von wasserlöslichen Arylaminen beispielsweise solchen sulfogruppenhaltigen der Benzol-oder Naphthalinreihe, die Substituenten wie Alkyl, Alkoxy, Halogen, Amino, Hydroxy and Carboxy aufweisen können, die Zwischenprodukte für die Herstellung faserreaktiver Farbstoffe sind.

Die nach dem erfindungsgemäßen Verfahren erhältlichen 2-Amino-4,6-difluortriazine werden im allgemeinen mit einem weiteren Mol Amin zum Endfarbstoff bzw, dem für den Endfarbstoff verwendeten Zwischenprodukt umgesetzt.

Besonders geeignete Ausgangsamine für das erfindungsgemäße Verfahren sind beispielsweise die folgenden:

1-Amino-8-hydroxy-naphthalin-3,6-disulfonsäure
1-Amino-8-hydroxy-naphthalin-4,6-disulfonsäure
2-Amino-5-hydroxy-naphthalin-7-sulfonsäure
2-Amino-8-hydroxy-naphthalin-6-sulfonsäure
2-Methylamino-5-hydroxy-naphthalin-7-sulfonsäure
1-Amino-8-hydroxy-naphthalin-4-sulfonsäure
1-Aminobenzol-3-sulfonsäure
1-Aminobenzol-4-sulfonsäure
1-Amino-4-methylbenzol-3-sulfonsäure
1-Amino-4-methoxybenzol-3-sulfonsäure
4-Aminobenzoesäure
1-Aminonaphthalin-6-sulfonsäure
2-Aminonaphthalin-5-sulfonsäure
2-Aminonaphthalin-6-sulfonsäure
2-Aminonaphthalin-8-sulfonsäure
1,3-Diaminobenzol-4-sulfonsäure
1,4-Diaminobenzol-2-sulfonsäure

2-Aminoethansulfonsäure

Besonders geeignet ist das erfindungsgemäße Verfahren für die Umsetzung von Cyanurfluorid mit 2-Amino-5-hydroxynaphthalin-7-sulfonsäure, 2-Amino-8-hydroxynaphthalin-6-sulfonsäure, 1-Amino-8-hydroxynaphthalin-4,6-disulfonsäure oder vor allem 1-Amino-8-hydroxynaphthalin-3,6-disulfonsäure wobei diese vorzugsweise in Form ihrer neutralen Alkalisalze (Li, Na, K) eingesetzt werden.

Vorzugsweise acyliert man in Wasser-Salz-Gemischen, die infolge Gefrierpunktserniedrigung für die Durchführung der Reaktion besonders geeignet sind.

Geeignete Salze sind insbesondere anorganische Salze wie Halogenide, Sulfate und Phosphate der Alkali- und Erdalkalimetalle, wie Lithiumchlorid, Natriumchlorid, Kaliumchlorid, Magnesiumchlorid, Calciumchlorid, Natriumsulfat, Kaliumsulfat, Magnesiumsulfat, Dinatriumhydrogenphosphat, Natriumdihydrogenphosphat, Kaliumdihydrogenphosphat, Dikaliumhydrogenphosphat, Natriumfluorid, Kaliumfluorid sowie Ammoniumchlorid oder Ammoniumsulfat.

Geeignete organische Salze sind beispielsweise quartäre Ammoniumsalze wie Tetra-alkylammoniumhalogenide, beispielsweise Tetramethylammonium-chlorid, Tetraethylammoniumchlorid oder Salze organischer Sulfosäuren, insbesondere solchen von Alkyl- und Arylsulfonaten, insbesondere $C_1$-$C_4$-Alkylsulfonaten und solchen der Benzol- und Naphthalinreihe, wobei als Kationen vorzugsweise Li, Na und K in Frage kommen. Besonders geeignet sind die Salze der Methan-, Benzol- oder Naphthalin-sulfosäuren (Mono-, Di- oder Trisulfosäuren).

Die Wahl und Menge des Salzes richtet sich dabei nach der Löslichkeit des zu acylierenden wasserlöslichen organischen Amins. Man kann auch Mischungen von Salzen verwenden. Die Menge kann in weiten Grenzen schwanken. Sie liegt im allgemeinen zwischen 1 und 30 Gew.-% bezogen auf das Gewicht der Reaktionslösung bzw. -suspension, vorzugsweise zwischen 2 und 15 Gew.-%, insbesondere zwischen 3 und 10 Gew.-%.

Als Beispiele für organische wasserlösliche Verbindungen seien folgende aufgeführt: Alkohole, wie Ethanol, Isopropanol, Ethylenglykol, Diethylenglykol, Ether wie Glykoldimethylether, Diethylenglykoldimethylether, Glykolmonomethylether, Butylglykol, Säureamide, wie Formamid, Dimethylformamid, Harnstoff, Tetramethylharnstoff, Caprolactam, N,N′-Dimethylharnstoff und Sulfolan. Auch hier kann die Menge in weiten Grenzen schwanken. Sie liegt im allgemeinen zwischen 1 % und 50 Gew.-%, bezogen auf das Gesamtgewicht der Reaktionslösung bzw. -suspension, vorzugsweise zwischen 5 und 30 Gew.-%, insbesondere zwischen 10 und 20 Gew.-%.

Während der Kondensation liegt die Temperatur unter $0^0$, vorzugsweise zwischen $-1^0$ und $-20^0$, insbesondere zwischen -2 und $-15^0$, insbesondere zwischen -4 und $-12^0$.

Diese Temperatur kann beispielsweise erreicht werden, indem man von außen mit einer Kühl-Sole kühlt. Man kann aber auch so verfahren, daß man ohne zusätzliche Kühlung die zu acylierende wasserlösliche organische Aminoverbindung in Wasser vorlegt, Salz hinzufügt und Eis einwirft, wobei eine Abkühlung auf unter $0^0$ erfolgt. Dieser Effekt reicht im allgemeinen aus um die Temperatur während der Acylierung unter $0^0$ zu halten.

Zur Neutralisation der freiwerdenden Flußsäure verwendet man als alkalisch reagierende Substanz insbesondere Oxide, Hydroxide, Hydrogencarbonate, Carbonate und Phosphate der Alkali- und Erdalkalimetalle; z.B. Lithiumhydroxid, Lithiumcarbonat, Natriumhydroxid, Natriumcarbonat, Natriumhydrogencarbonat, Kaliumhydroxid, Kaliumcarbonat, Magnesiumhydroxid, Magnesiumcarbonat, Calciumoxid, Calciumhydroxid, Calciumcarbonat. Im Prinzip ist auch ein tertiäres aliphatisches Amin geeignet, wie Triethylamin oder Triethanolamin.

Die angegebenen Formeln sind die der freien Säuren, auf die sich auch die Gewichtsangaben beziehen. Die aromatischen Sulfosäuren liegen im allgemeinen als Salze vor, insbesondere der Natrium-, Kalium- oder Lithiumsalze.

Bei den Temperaturangaben handelt es sich um $^0C$.

## Beispiel 1

46,2 g H-Säure = 1-Amino-8-hydroxy-naphthalin-3,6-disulfonsäure (Mono-Natriumsalz) werden in 120 ml Wasser mit 30 ml 11 %iger Lithiumhydroxidlösung gelöst, wobei sich ein pH von 6,0 bis 6,5 einstellt. Diese Lösung (190 ml) gießt man in ein Dewargefäß und gibt 19 g Lithiumchlorid zu wobei die Temperatur auf $38^0$ ansteigt. Anschließend setzt man 10 g Borsäure zu. Durch Einwerfen von 300 g Eis erniedrigt sich die Temperatur auf $-8^0$. Man tropft nun in 20 Minuten 19,6 g Trifluortriazin ein, wobei man zunächst den pH von 6,0 bis 6,5 auf 4,3 abfallen läßt und nach Erreichen dieses Wertes den pH durch Eintropfen von Lithiumhydroxidlösung zwischen 4,0 und 4,5 hält. Während der Acylierung tritt ein Niederschlag auf, der

gegen Ende der Acylierung wieder in Lösung geht. Man prüft nun auf vollständige Acyierung der H-Säure und setzt erforderlichenfalls noch 1-2 g Trifluortriazin zu. Nach beendeter Acylierung beträgt die Reaktionstemperatur -5°. Anschließend tropft man 13,5 g Anilin zu, wobei die Temperatur über 0° ansteigt. Den pH stellt man durch Zugabe von Lithiumhydroxidlösung auf 5,5 und führt die Kondensation mit Anilin bei einer Temperatur von 0 bis 10° zu Ende. Das Kondensationsprodukt ist zum Teil ausgefallen. Es entspricht folgender Formel

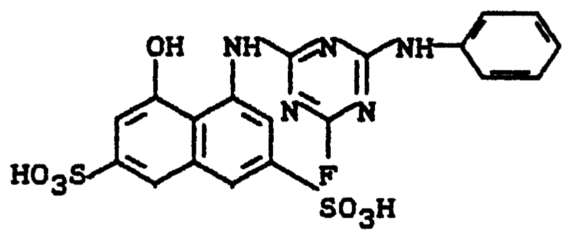

Es liegt als gemischtes Lithium-Natriumsalz vor.

Zu der erhaltenen Suspension gibt man eine Diazoniumsalz-Suspension von 0 bis 5°, welche durch Diazotierung auf übliche Weise von 0,145 Mol 2-Aminonaphthalin-1,5-disulfonsäure erhalten wurde. Bei maximal 10° wird im pH-Bereich von 6 bis 7,5 ausgekuppelt.

Man erhält den Farbstoff der Formel

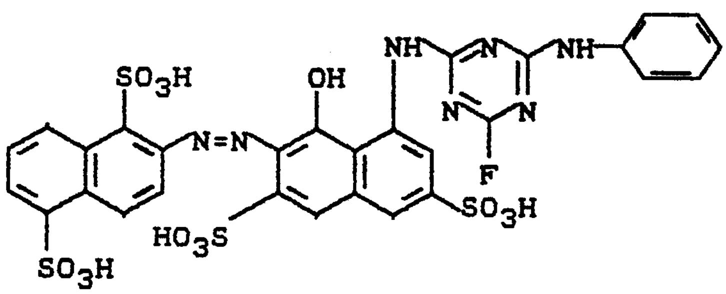

in Form des gemischten Li-Na-Salzes in guter Ausbeute. Er färbt Baumwolle in waschechten klarem Rotton.

Nach den bisher üblichen Verfahren, d.h. bei Durchführung der Kondensation von Cyanurfluorid mit 1-Amino-8-hydroxynaphthalin-3,6-disulfonsäure bei 0-5° C erhält man bei im übrigen gleicher Arbeitsweise den vorstehend beschriebenen Farbstoff in deutlich geringerer Ausbeute, der zudem noch mit roten Nebenprodukten verunreinigt ist.

<u>Beispiel 2</u>

46,2 g H-Säure = 1-Amino-8-hydroxy-naphthalin-3,6-disul-fonsäure (Mono-Natriumsalz) werden in 150 ml Wasser mit 30 ml 11 %iger Lithiumhydroxidlösung gelöst, wobei sich ein pH von 6,0 bis 6,5 einstellt. Diese Lösung versetzt man mit 22 g Lithiumchlorid, wobei sie sich auf 35-40° C erwärmt. Dann erniedrigt man den pH durch Zutropfen von verd. Salzsäure auf 4,8 und kühlt anschließend von außen mit einer Kühl-Sole auf -10° C ab. Man tropft nun bei -8° bis -11° C in 20 Minuten 19,6 g Trifluortriazin ein, wobei man den pH zunächst von 4,8 auf 4,0 abfallen läßt und nach Erreichen dieses Wertes ihn durch Eintropfen von Lithiumhydroxidlösung zwischen 4,0 und 4,5 hält. Während der Acylierung tritt ein Niederschlag auf, der gegen Ende der Acylierung wieder in Lösung geht. Man prüft nun auf vollständige Acylierung der H-Säure und setzt erforderlichenfalls noch 1-2 g Trifluortriazin zu. Nach beendeter Acylierung beträgt die Reaktionstemperatur -10°. Anschließend tropft man 13,5 g Anilin zu, wobei die Temperatur über 0° ansteigt. Den pH stellt man durch Zugabe von Lithiumhydroxidlösung auf 5,5 und führt die Kondensation mit Anilin bei einer Temperatur von 0 bis 5° zu Ende. Das Kondensationsprodukt ist zum Teil ausgefallen. Es entspricht folgender Formel

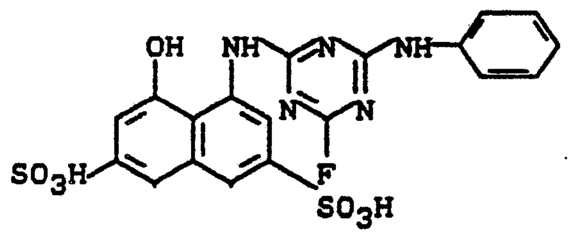

Es liegt als gemischtes Lithium-Natriumsalz vor.

Durch Diazotieren von 2-Amino-naphthalin-1,5-disulfonsäure und Kuppeln erhält man gleichfalls in guter Ausbeute den in Beispiel 1 beschriebenen Farbstoff.

## Ansprüche

1. Verfahren zur Monoacylierung von wasserlöslichen organischen Aminen mit 2,4,6-Trifluortriazin in wäßrigem Medium, dadurch gekennzeichnet, daß man die Reaktion bei Temperaturen unter 0 °C in Gegenwart anorganischer und/oder organischer wasserlöslicher Salze und/oder wassermischbarer organischer Lösungsmittel durchführt.

2. Verfahren nach Anspruch 1, dadurch gekennzeichnet, daß man die Reaktion bei -1 bis -20 °C durchführt.

3. Verfahren nach Anspruch 1, dadurch gekennzeichnet, daß man die Reaktion bei -2 bis -15 °C durchführt.

4. Verfahren nach Anspruch 1, dadurch gekennzeichnet, daß man die Reaktion bei -4 bis -12 °C durchführt.

5. Verfahren nach Ansprüchen 1-4, dadurch gekennzeichnet, daß als wäßriges Medium ein Gemisch aus Wasser und anorganischem Salz verwendet wird.

6. Verfahren nach Ansprüchen 1-5, dadurch gekennzeichnet, daß als Amin aromatische Aminosulfosäuren verwendet werden.

7. Verfahren nach Anspruch 6, dadurch gekennzeichnet, daß man als Amin 1-Amino-8-hydroxynaphthalin-3,6-bzw. -4,6-disulfosäure, 2-Amino-5-hydroxynaphthalin-7-sulfosäure oder 2-Amino-8-hydroxynaphthalin-6-sulfosäure verwendet.

Europäisches Patentamt

# EUROPÄISCHER RECHERCHENBERICHT

Nummer der Anmeldung

EP 88 11 2588

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 094 538 (BAYER AG)<br>* Anspruch 7; Seite 22, Beispiel B-1 * | 1-5 | C 07 D 251/50 |
| D,A | DE-A-2 903 594 (BAYER AG)<br>* Anspruch 1; Beispiele 1,2 * | 1,7 | |
| D,A | EP-A-0 172 790 (CIBA-GEIGY AG)<br>* Ansprüche 1,2; Beispiele 1,2 * | 1,7 | |
| D,A | DE-A-2 747 011 (CIBA-GEIGY AG)<br>* Anspruch 1; Beispiel 1 * | 1,7 | |
| D,A | DE-A-2 746 109 (CIBA-GEIGY AG)<br>* Ansprüche 1,3 * | 1,7 | |
| A | EP-A-0 036 133 (HOECHST AG)<br>* Beispiele 6-8 * | 1,7 | |
| A | DE-A-2 901 498 (CIBA-GEIGY AG)<br>* Anspruch 1 * | 1 | |

RECHERCHIERTE SACHGEBIETE (Int. Cl.4)

C 07 D 251/00

Der vorliegende Recherchenbericht wurde für alle Patentansprüche erstellt

| Recherchenort | Abschlußdatum der Recherche | Prüfer |
|---|---|---|
| BERLIN | 09-11-1988 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)